Europäisches Patentamt

European Patent Office

Office européen des brevets

(11) Publication number: **0 330 479**
A2

## EUROPEAN PATENT APPLICATION

(21) Application number: **89301789.7**

(51) Int. Cl.⁴: **C 12 N 15/00**

(22) Date of filing: **23.02.89**

(30) Priority: **26.02.88 US 160818**

(43) Date of publication of application:
**30.08.89 Bulletin 89/35**

(84) Designated Contracting States:
**AT BE CH DE ES FR GB GR IT LI LU NL SE**

(71) Applicant: **LUBRIZOL GENETICS INC.**
**29400 Lakeland Boulevard**
**Wickliffe Ohio 44092 (US)**

**THE UNIVERSITY OF FLORIDA**
**223 Grinter Hall**
**Gainesville Florida 32611 (US)**

(72) Inventor: **Gurley, William B.**
**2001 S.W. 16th Street Apartment A3**
**Gainesville Florida 32608 (US)**

**Czarnecka, Eva**
**2307 S.W. 32nd Place**
**Gainesville Florida 32608 (US)**

**Mosquera, Luis**
**3010 S.W. 23rd Terrace**
**Gainesville Florida 32608 (US)**

(74) Representative: **Fisher, Adrian John et al**
**CARPMAELS & RANSFORD 43 Bloomsbury Square**
**London WC1A 2RA (GB)**

(54) **Plant universal stress-inducible regulatory element.**

(57) A plant universal stress-inducible regulatory element comprises 5′-flanking DNA sequences of a plant gene which exhibits induced synthesis in response to a variety of environmental stresses, e.g., heat shock, osmotic pressure, salt stress, heavy metal ion concentration, partial anaerobiosis, etc. The isolation, characterization and utility of such a regulatory element is disclosed. The plant universal stress-inducible regulatory element contains promoter functionality, associated with sites of initiation of transcription, as well as regulatory function associated with the induction response to a variety of stresses. Methods are provided for inducing an increased level of expression of plant-expressible genes in plant tissue in response to environmental stress.

FIG. 1

## Description

## PLANT UNIVERSAL STRESS-INDUCIBLE REGULATORY ELEMENT

### FIELD OF THE INVENTION

The field of this invention is the area of plant molecular biology in general, and relates specifically to plant genetic engineering by recombinant DNA technology. This invention relates to the isolation, characterization, and use of 5'-flanking DNA sequences of a plant gene which exhibits induced synthesis in response to a variety of environmental stresses. These sequences constitute a plant universal stress-inducible regulatory element which will enable the induced expression in plants of desired structural genes after exposure to environmental stresses including, but not limited to, elevated temperatures (heat shock), water stress, salt stress, heavy metal ions, partial anaerobiosis, certain respiratory inhibitors, amino acid analogs, nonphysiologically high levels of plant growth regulators such as abscisic acid or 2,4-diphenoxyacetic acid, and wounding.

### BACKGROUND OF THE INVENTION

In eukaryotic genes there is a growing understanding of the DNA sequence elements which control gene expression. The following discussion applies to genes which are transcribed by RNA polymerase II. There are known sequence elements which direct the initiation of mRNA synthesis, those which control transcription in response to environmental stimuli, and those which modulate the level of transcription.

Promoters are the portions of DNA sequence, at the beginnings of genes, which contain the signals for RNA polymerase to begin transcription so that protein synthesis can then proceed. Eukaryotic promoters are complex, and are comprised of components which include a TATA box consensus sequence in the vicinity of about -30, and often a CAAT box consensus sequence at about -75 bp 5' relative to the transcription start site, which is defined as +1 (R. Breathnach and P. Chambon (1981), Ann. Rev. Biochem. 50:349; J. Messing et al. (1983), in Genetic Engineering of Plants, eds. T. Kosuge, C. Meredith, and A. Hollaender, pp. 211). In plants there may be substituted for the CAAT box a consensus sequence which Messing et al. (1983) have termed the AGGA box, positioned a similar distance from the cap site. Other sequences in the 5'-untranscribed regions of genes are known which regulate the expression of the downstream genes. There are sequences which participate in the response to environmental conditions, such as illumination, nutrient availability, hyperthermia, anaerobiosis, or the presence of heavy metals. There are also signals which control gene expression during development, or in a tissue-specific fashion. Promoters are usually positioned 5' to, or upstream of, the start of the coding region of the corresponding gene, and the DNA tract containing the promoter sequences and the ancillary sequences affecting regulation or absolute levels of transcription may be comprised of less than 100 bp or as much as 1 kbp.

As defined by G. Khoury and P. Gruss (1983), Cell 33:313, an enhancer is one of a set of eukaryotic promoter-associated elements that appears to increase transcriptional efficiency in a manner relatively independent of position and orientation with respect to the nearby gene. The prototype enhancer is found in the animal virus SV40. Generally animal or animal virus enhancers can function over a distance as much as 1 kbp 5', in either orientation, and can act either 5' or 3' to the gene. The identifying sequence motif is typically reiterated. There have been sequences identified in plant genes which have homology to the core consensus sequence of the SV40 enhancer, but the functional significance of these sequences in plants has not been determined.

There are also reports of enhancer-like elements 5' to certain constitutive and inducible genes of plants. One such report is that of J. Odell et al. (1985), Nature 313:810; a stretch of about 100 bp of the 5'-nontranscribed region of the 35S gene of Cauliflower Mosaic Virus (CaMV) is necessary for increasing the expression of a reporter gene. Related reports state that tandem duplication of a portion of the upstream region causes further enhancement of reporter gene activity (R. Kay et al. (1987) Science 236:1299; D. Ow et al. (1987) Proc. Nat. Acad. Sci. USA 84:4870). Two unique transcription activating elements which function in plants are derived from the 780 gene and the octopine synthase gene of Agrobacterium tumefaciens T-DNA (W. Bruce and W. Gurley (1987) Mol. Cell. Biol. 7:59; W. Bruce and W. Gurley, Proc. Natl. Acad. Sci. USA, submitted (1987); J. Ellis et al. (1987) EMBO J. 6:11). Regulated enhancer-like elements include those believed to mediate tissue specific expression and response to illumination (M. Timko et al. (1985) Nature 318:579; H. Kaulen et al. (1986) EMBO J. 5:1; J. Simpson et al. (1985) EMBO J. 4:2723; J. Simpson et al. Nature 323:551; R. Fluhr et al. (1986) Science 232:1106).

Plants respond to elevated temperatures in a manner similar to that of bacteria, yeast, insects or mammals. The synthesis of normal cell protein stops, or is dramatically reduced, and the preferential transcription and translation of a relatively small set of proteins ensues. Those proteins whose synthesis is induced by hyperthermia are known as the heat shock proteins (HSPs). The size distributions of the HSPs are similar in the eukaryotes, and there are significant amino acid homologies in analogous proteins of different species (reviewed by S. Linquist (1986) Ann. Rev. Biochem. 55:1151). The best studied eukaryotic heat shock system is Drosophila (N. Ashburner and J. Bonner (1979) Cell 17:241).

In soybeans, heat shock elicits the synthesis of a characteristic set of HSPs, which can be divided into two classes. The low molecular weight HSPs range in size from 15 to 27 kDa; there are an estimated 30 to 50 members of this class. There are several members of the high molecular weight class of the soybean HSPs, ranging in size from 68 to 110 kDa (J. Key et al. (1983) in Curr. Top. in Plant Biochem. and Physiol., vol. 2, D. Randall et al., eds, University of Missouri-Columbia, Columbia, Missouri; J. Key et al. (1981) Proc. Nat. Acad. Sci. USA 79:3526). The exact functions of the HSPs are not known, but prior induction of these genes is correlated with the acquisition of thermotolerance in soybean seedlings (C.-Y. Lin et al. (1984) Plant Physiol. 74:152).

In plants, as in many other organisms, some of the HSPs are also induced to varying extents by other environmental stresses such as osmotic stress, wounding, exposure to heavy metals, and treatment with respiratory inhibitors, amino acid analogues, and the growth regulators 2,4-dichlorophenoxyacetic acid (2,4-D), or abscisic acid (E. Czarnecka et al. (1984) Plant Mol. Biol. 3:45; J. Heikkila et al. (1984) Plant Physiol. 76:270). It has been postulated that the common signal which leads to the induction of the HSPs in response to these stresses is abnormal or damaged cellular protein (J. Ananthan et al. (1986) Science 232:522). Expression of each soybean heat shock gene is not induced by all environmental stresses; therefore there may be no single induction mechanism controlling the response to all of the stresses which elicit heat shock gene expression (E. Czarnecka et al. (1984)). Furthermore, some heat shock proteins show detectable levels of constitutive expression, and in other systems (e.g. Drosophila), some heat shock genes are activated at specific times during development. It is not yet known whether the regulatory regions of plant heat shock genes possess properties of inducible enhancers. The untranslated leader region of the heat shock mRNA may also possess regulatory information which affects the relative efficiency of the synthesis of the heat shock gene products (M. Scott and M. Pardue (1981) Proc. Natl. Acad. Sci. USA 78:3353; R. Storti et al., (1980) Cell 22:825).

Other environmental stresses may induce other sets of genes; for example, anaerobiosis in plants induces the synthesis of several major proteins (R. Okimoto et al. (1980) Planta 150:89). The functions of several of these proteins have been established; these enzymes participate in the change in energy metabolism necessary to adapt to the absence of oxygen. (M. Freeling et al. Ann. Rev. Genet. 19:297). Exposure of Antirrhinum majus to UV light induces the synthesis of chalcone synthase, an enzyme which catalyzes a key step in the synthesis of a protective pigment (H. Kaulen et al. EMBO J. 5:1). Among the eukaryotes the induction of metallothionein is a common response to exposure to heavy metal ions in the environment. The metallothioneins are small cysteine-rich, metal binding proteins, and as such, are believed to offer some protection against heavy metal poisoning (reviewed by D. Hamer (1986) Ann. Rev. Biochem. 55:913).

There have been several sequences, located in the 5'-flanking regions of plant genes, which have been proposed as plant regulatory elements: it is postulated that these elements control tissue specificity and responses to environmental conditions such as light and anaerobiosis. For example, fragments of DNA from the 5'-untranscribed regions of ribulose bisphosphate carboxylase subunits and chlorophyll binding protein genes have been described which carry sequence information conferring light regulation to these genes and which exhibit some properties of enhancer-like elements (R. Fluhr et al. (1986) Science 232:1106; J. Simpson et al. (1985) EMBO J. 4:2723; J. Simpson et al. (1986) Nature 323:551; M. Timko et al. (1985) Nature 318:579). Plant DNA regulatory sequences have been characterized which confer anaerobic inducibility on the alcohol dehydrogenase genes of maize on heterologous plant-expressible genes (J. Walker et al. (1987), Proc. Natl. Acad. Sci. USA 84:6624-6628).

Regulatory metal response elements (MREs) have been identified in the 5'-flanking regions of mammalian genes encoding metallothioneins, proteins which tightly bind heavy metal ions, and whose synthesis is induced by exposure to heavy metal ions. Multiple copies of the MREs placed 5' of heterologous genes confer metal regulation on those genes. Moreover, the MREs act as inducible enhancers of transcription when located either 5' or 3' to heterologous genes. The consensus sequences for the mammalian MRE is 5'-CPyTTTGCPuPyPyPyCG-3' wherein Pu is A or G and Py is C or T (G. Stuart et al. (1984) Proc. Nat. Acad. Sci. USA 81:7318). There appear to be no reports concerning specific DNA sequences which regulate response to heavy metal ions in plants.

The sequence motifs, termed heat shock elements (HSEs), which direct the induction of the heat shock genes in response to the stress of elevated temperature, have been studied in bacteria, yeast, insects, man and plants. In Drosophila the consensus sequence for the motif is 5'-CTxGAAxxTTCxAG-3' wherein x is A, T, C, or G (H. Pelham (1982) Cell 30:517). The Drosophila HSEs bind a protein factor necessary for the activation of downstream heat shock gene expression during thermal stress (C. Parker and J. Topol (1984) Cell 37:273). Generally an 8 out of 10 match to the consensus sequence is required for HSE functionality, but it has been shown that poorer matches are active when reiterated (A. Ayme et al. (1985) J. Mol. Biol. 182:469; M. Bienz (1985) Trends Biochem. Sci. 10:157; H. Pelham (1982) EMBO J. 1:1473). The HSEs of Drosophila also exhibit some properties of enhancer elements (M. Bienz and H. Pelham (1986) Cell 45:753).

There are several reports concerning the expression of heat shock genes in heterologous systems. H. Pelham et al. (1982), described the heat-induced transcription of Drosophila hsp70 in SV40-transformed monkey COS cells. Similar observations were made by M. Mirault et al. (1982) EMBO J. 1:1279 in that transcription was induced by heat shock or by arsenite treatment. Heat-regulated transcription of Drosophila heat shock genes was also reported in Xenopus oocytes (H. Pelham and M. Bienz (1982) EMBO J. 1:1473; R. Voellmy and D. Runnger (1982) Proc. Nat. Acad. Sci. 79:1776). A divergently transcribed pair of hsp16 genes of the nematode Caenorrhabditis elegans was transformed into a mouse fibroblast cell line; transcription was dependent of stimulation by heat shock or arsenite treatment (R. Kay et al (1986) Mol. Cell. Biol. 6:3134). The results of that study suggest that the number of HSEs can be a determinant of promoter strength; one HSE between the divergent TATA elements gave regulated transcription at about 10% of wild type levels, but four overlapping HSEs between the TATAs gave greater than wild type levels. It is, however, not yet explicitly known how the arrangement and spacing of HSEs affect promoter activity.

Heterologous expression of heat shock genes has also been documented in plants. The expression of a soybean gene predicted to encode a 17.3 kDa heat shock protein (hs6871) was studied in sunflower hypocotyl tumor tissue after T-DNA-mediated transformation (F. Schoffl and G. Baumann (1985) EMBO J. 4:1119). The approximately 1 kb of 5'-flanking DNA sequence contained sufficient information to regulate heat-induced gene expression. W. Gurley et al. (1986) Mol. Cell. Biol. 6:559, also observed regulated transcription of a small heat shock gene of soybean in transformed sunflower tissue. Transcription was induced by arsenite and cadmium treatments as well as by heat shock. D. Rochester et al. (1986) EMBO J. 5:451, studied the expression of a hybrid maize hsp70 gene in transformed petunia leaves. Induction of expression by heat shock was maintained in the heterologous host plant, therefore it was inferred that the transcription was directed by the heat shock-responsive sequence elements contained within the 1.1 kb of 5'-flanking DNA sequences.

Recombinant DNA molecules have been constructed in which the promoter and associated DNA sequences from a heat shock gene direct regulated transcription of a heterologous gene. M. Bienz and H. Pelham (1986) Cell 45:753, demonstrated that upstream sequences from the Xenopus hsp70 gene control the expression of a globin reporter gene in a mammalian cell line. Drosophila heat shock promoters have been used to control the expression of reporter genes in mammalian systems as well (A. Ayme et al. (1985); D. Pauli et al. (1986) EMBO J. 5:755). A. Spena et al. (1985) EMBO J. 4:2739, reported that Drosophila heat shock regulatory sequences function correctly in plant tissue. The regulation of heat shock genes in plants appears to be similar to the regulation of those genes in other organisms. D. Rochester et al. (1986) surmised that the 1 kb 5' to the start of the transcribed region was sufficient to correctly control heat shock- dependent transcription. In the report by A. Spena et al. (1985), 258 bp of Drosophila promoter sequence was sufficient to give regulated transcription in transformed tobacco tissue, but no attempt to determine the effects of longer or shorter promoter fragments was reported. W. Gurley et al. (1986), described sequence elements with partial homology to the Drosophila HSE consensus sequence at the 5'-end of the soybean Gmhsp17.5-E gene. The study of expression in transformed sunflower tumor tissue revealed that DNA sequence information between -95 and the cap site was sufficient to direct thermoinducible transcription, but that sequences between -95 and -1175 dramatically increased both induced and basal levels of transcription.

## SUMMARY OF THE INVENTION

The work of the present invention describes the isolation and characterization of a universal stress-inducible regulatory element, a DNA sequence functional in plants, which regulates the expression of downstream structural genes in response to the application of a wide variety of environmental stresses, including, but not limited to heat shock, exposure to heavy metal ions, partial anaerobiosis, osmotic stress, salt stress, and treatment with certain respiratory inhibitors, acid analogues, or plant growth regulators. This regulatory element is unique in that it exhibits an inductive response to many more environmental stresses than do other known plant heat shock regulatory sequences.

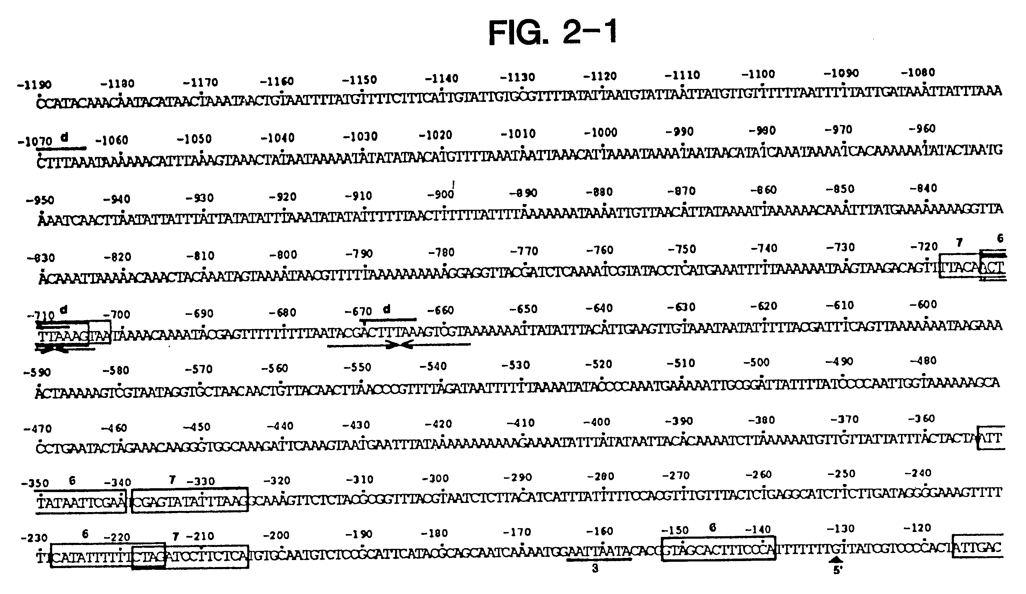

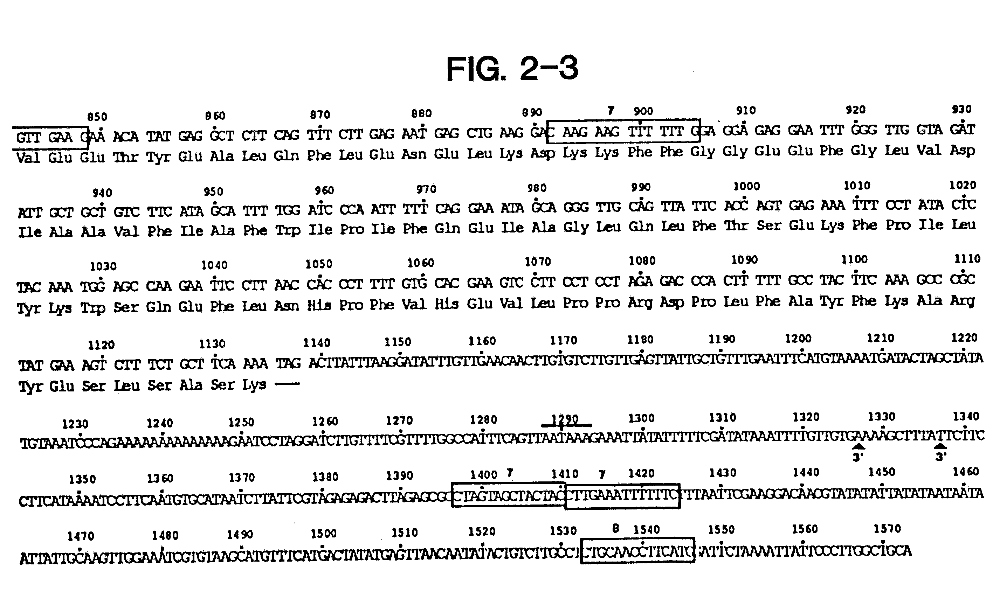

A specific example of such a plant regulatory element is found in the 5'-upstream region of the Gmhsp26-A gene of soybean, the sequence of which is given in Figure 2. Analysis of the nucleotide sequence of the 5'-flanking regions of this gene indicates that the sequence information necessary to correctly regulate stress-induced expression and initiate transcription and translation of a structural gene is contained within the region extending 5' to about position -720 as measured from the initiation site of the major transcript. Other sequences which contribute to the level of expression of a downstream gene can be found in the region upstream of -720 to about -1190 and within the region comprising the 5'-untranslated portion of the gene (about +1 to about +72). The Gmhsp26-A gene universal stress-inducible regulatory element contains three overlapping promoter regions, indicated by the presence of TATA box homologies. The untranscribed region of the gene contains ten sequences which each have 50% or greater homology to the consensus heat shock element. These plant heat shock elements occur within the region extending from about -35 to about -720. Sequences homologous to known metal regulatory response elements are also found within this DNA region. Such sequences can represent sites of binding of regulatory factors to DNA. One or more of the plant heat shock sequences is believed to function in stress-response. There is an additional sequence having about 70% homology to the consensus heat shock element in the 5'-untranslated region of the gene at about +45. Sequences in the 5'-untranslated region extending to the translation initiation codon (ATG) can contribute to the efficiency of gene expression.

A primary object of this invention is to enable those skilled in the art to achieve selectively enhanced expression of structural genes in plants in response to conditions of a variety of stresses. This object is accomplished by utilizing a DNA sequence, designated the plant universal stress-inducible regulatory element. This sequence confers induction by a very wide variety of environmental stress conditions. The universal stress-inducible regulatory element contains promoter sequences as well as promoter-associated regulatory sequences, which function in the regulation of gene expression. The DNA sequence which constitutes the universal stress-inducible regulatory element contains one or more sequence motifs which have 50% or greater homology to the consensus heat shock element. Such sequence motifs represent plant heat shock elements.

In a specific embodiment, the universal stress-inducible regulatory element of the Gmhsp26-A gene is provided. The DNA sequence extending from about -720 to -21 (as in Figure 2), of the Gmhsp26-A gene functions as a plant universal stress-inducible regulatory element. This DNA sequence, -720 to -21, includes three overlapping promoters, as indicated by the presence of TATA box homologies as well as all of the plant heat shock elements found in the nontranscribed region of the gene. DNA fragments of the Gmhsp26-A gene that will function as plant universal stress-inducible regulatory elements include those fragments extending from about -720 to about +1 (the major transcription initiation site), from about -720 to about +72 (translation initiation site), from about -1190 to about -21, from about -1190 to about +1 and from about -1190 to about +72. Use of the sequence extending from -1190 to +72 is preferred, since all ancillary sequence which can contribute to effective stress-induced expression are included therein. DNA fragments having at least about 90% homology to the Gmhsp26-A fragments which function as universal stress-inducible regulatory elements are considered to be functionally equivalent thereto.

The invention provides recombinant DNA molecules which comprise a plant universal stress-inducible regulatory element and a plant-expressible structural gene, wherein the structural gene is positioned 3' to the regulatory element and under its regulatory control with the result that increased levels of expression of the structural gene are obtained on application of stress conditions. Specifically, DNA molecules containing the universal stress-inducible regulatory element of the Gmhsp26-A gene are provided. It is preferred that the DNA molecules of the present invention comprise structural genes which are not those of plant heat shock genes.

The DNA molecules of the present invention are useful in a method for selectively inducing an increased level of expression of a desired plant-expressible structural gene in a plant cell in response to the application of a wide variety of environmental stresses. The method of the present invention comprises introducing into a plant cell a recombinant DNA molecule which contains a plant universal stress-inducible regulatory element and the desired plant-expressible structural gene which is positioned such that it is under the regulatory control of the stress-inducible regulatory element. Subsequent application of stress conditions to the transformed plant cell results in stress-induced expression of the desired structural gene. The method of the present invention is generally applicable to selective gene expression in both dicotyledonous and monocotyledonous plants and can in general be applied to the expression of any structural gene that can be expressed in a plant. The method is particularly useful for the expression of structural genes which are not plant heat shock structural genes.

The DNA molecules described herein can be introduced into plant tissue so that the expression of the stress-inducible regulatory element/structural gene combination can be selectively increased by application of stress conditions in that tissue. Transformation of plant cells and tissue with exogenous or foreign DNA can be achieved by any means known to the art. In a specific embodiment, this was achieved by T-DNA-mediated transformation.

Other objects of this invention are plants, plant cells and plant tissue containing the recombinant DNA molecules and chimeric stress-inducible genes described herein and prepared by the methods described herein.

BRIEF DESCRIPTION OF THE FIGURES

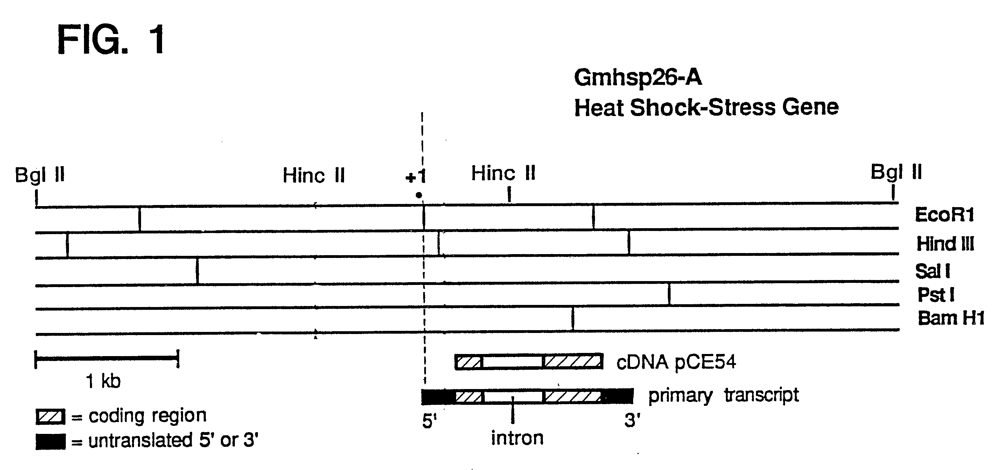

Figure 1 displays a restriction endonuclease map of the 6.2 kb BglII fragment of the Glycine max genome which contains the Gmhsp26-A gene plant universal stress-inducible regulatory element. The position of the Gmhsp26-A gene primary transcript is indicated, along with that of the cDNA pCE54. The location of the intron of Gmhsp26-A is indicated; the gene coding region is indicated by cross-hatching and the 5'- and 3'-untranslated regions of the gene are indicated by dark shading. The position of the major transcription start of the gene is indicated as +1.

Figure 2 displays the DNA sequence of the Gmhsp26-A gene, from -1190 to +1573. The amino acid sequence deduced from the coding sequence is displayed below the nucleotide sequence. Three transcription start sites are marked by solid triangles beneath the nucleotide sequence, and the start site of the major transcript is identified by a superscript numeral 1. The TATA box-like sequence motifs corresponding to subpromoters 1, 2, and 3 are underlined and labelled with the corresponding numerals. DNA sequences with homology to the consensus heat shock element are boxed, and the number of bases (out of 10) which match the consensus are given over each box. Direct repeats are overlined and labelled with the letter "d"; indirect repeated sequences are underscored with arrows. The polyadenylation signal in the 3' region of the Gmhsp26-A gene is overlined.

DETAILED DESCRIPTION OF THE INVENTION

The following definitions are provided, in order to remove ambiguities to the intent or scope of their usage in the specification and claims.

The Gmhsp26-A gene described herein is a heat shock gene of soybean. It is identified by the DNA sequence presented in Figure 2, and it is predicted to encode a protein of about 26 kDa. Gmhsp26-A has a detectable level of constitutive expression and it undergoes induction to higher expression levels when the plant tissue is exposed to a variety of conditions of environmental stress, including, but not limited to, elevated temperatures (heat shock), osmotic stress, salt stress, heavy metal ions, partial anaerobiosis, certain respiratory inhibitors, amino acid analogs, nonphysiologically high levels of plant growth regulators such as abscisic acid or 2,4-diphenoxyacetic acid, and wounding. The fact that this gene is induced in response to such a variety of environmental stimuli distinguishes it from other known heat shock genes of plants.

The term plant universal stress-inducible regulatory element refers herein to a functional DNA sequence which, when placed upstream of a plant-expressible structural gene, can direct the stress-induced expression of that structural gene. The environmental stresses which can elicit induction of the structural gene under the regulatory control of the plant universal stress-inducible regulatory element include, but are not limited to, elevated temperatures (heat shock), osmotic stress, salt stress, heavy metal ions, partial anaerobiosis, certain respiratory inhibitors, amino acid analogs, nonphysiologically high levels of plant growth regulators such as abscisic acid or 2,4-diphenoxyacetic acid, and wounding. The broad range of environmental stresses which elicit induction of gene expression by the stress-inducible regulatory element is what distinguishes this regulatory element from others associated with known plant heat shock genes. The plant universal stress-inducible regulatory element contains promoter functionality, associated with sites of initiation of transcription, as well as regulatory function associated with the induction response to stress. The universal stress response of this regulatory element is associated with the presence of a plurality of discrete relatively short regulatory sequences, ranging in size up to about 20 nucleotides in length. These discrete regulatory sequences function in the induction of gene expression in response to application of stress, for example by acting as binding sites for regulatory proteins. Such regulatory sequences can either be responsive to a single stress stimulus or to multiple stress stimuli. A single isolated regulatory sequence can be required for inducive response or the coordinate functioning of two or more of such sequences can be required for induction response. Plant heat shock element homologs are functional in the regulation of stress induction by the plant universal stress-inducible regulation element. The plant universal stress-inducible regulatory element of the present invention is exemplified by that of the Gmhsp26-A gene of soybean.

The 5'-region of the Gmhsp26-A gene extends from about nucleotide -1190 to about nucleotide +72 (i.e., to the translation start site), as in Figure 2. Included within this region are promoter-associated sequences which function to induce gene expression in response to stresses such as heat shock or heavy metal ions and promoter sequences which direct the initiation of transcription. The region extending 5' from about -21 (the location of the TATA box of the major transcription start) to about -720 is sufficient to give stress-inducible regulation of a downstream gene. Sequences in the region between about -1190 to -720 and in the 5'-untranslated region (about +1 to about +72) can contribute to efficiency of expression. In the 5'-flanking region of the Gmhsp26-A gene there are ten sequences with at least 50% homology to the consensus heat shock element and three TATA-homologous sequences are found between about nucleotide -720 and about -21, as in Figure 2. An eleventh heat shock element-homologous sequence is found in the untranslated leader region of the mRNA at around +45, between the cap site and the translation start site at about +72. One or more of these heat shock homologous elements function in the response to application of stress. Three subpromoters were identified in the Gmphsp26-A gene 5'-flanking region which display somewhat differential response to stress stimuli. The Gmhsp26-A universal stress-inducible regulatory element contains discrete regulatory sequences in addition to heat shock element homologs which function in the induction of expression in response to stress stimuli. The locations of such functional nucleotides in the regulatory element sequences can be determined using conventional techniques in which the functionality of mutant derivatives of the regulatory region (i.e., 5'-deletion mutations, internal deletion mutations, and site-specific mutations) for gene expression are assessed (see Example 7). Such techniques have been successfully applied to the analysis of regulatory regions (as for example in Bruce and Gurley (1988) Proc. Natl. Acad. Sci. USA, submitted, and in U.S. Application Serial No. 135,147, filed December 18, 1987)[1].

Expression refers to the transcription and translation of a structural gene so that a protein is made. Gene expression may be assessed by direct detection of the protein product, by protein electrophoresis or by immunological methods, for example. Alternatively, expression may be assessed by detection of the mRNA products of transcription (e.g. by Northern hybridizations). This method is particularly appropriate for the testing of transcriptional regulatory sequences because the effects of posttranscriptional processes such as protein degradation are excluded.

Promoter refers to the DNA sequences at the 5'-end of a structural gene which direct the initiation of transcription. Promoter sequences are necessary, but not always sufficient, to drive expression of the downstream structural genes. The promoter itself may be a composite of segments derived from more than one source, naturally occurring or synthetic. Eukaryotic promoters are commonly recognized by the presence of DNA sequence element homologous to the canonical form 5'-TATAAT-3' (TATA box) about 10-30 bp 5' to the 5'-end of the mRNA (cap site, +1). About 30 bp 5' to the TATA box another promoter component sequence is often, but not always, found which is recognized by the presence of DNA sequences homologous to the canonical form 5'-CCAAT-3'. For the purposes of this application, a promoter is considered to extend about 100 bp 5' of the transcription start site. In the case of the Gmhsp26-A gene, there are three overlapping promoters. Promoter-associated sequences located further upstream from about -100 may contribute to, or exert, regulatory control and may determine relative levels of gene expression. There may also be sequence elements which contribute to gene regulation at either the transcriptional or the translational level in the region between -100 and the translation start site.

Structural gene refers to that portion of a gene comprising a DNA segment coding for a protein, polypeptide

[1] and European Patent Application No. 88311807.7

or portion thereof, possibly including a ribosome binding site and/or a translational start codon, but lacking at least one component of 5'-sequence which drives the initiation of transcription. The term can also refer to copies of a structural gene naturally found within the cell but artificially introduced. The structural gene may encode a protein not normally found in the plant cell in which the gene is introduced, in which case it is termed a foreign structural gene. A foreign structural gene may be derived in whole or part from a bacterial genome or episome, eukaryotic nuclear or plastid DNA, cDNA, viral DNA, or chemically synthesized DNA. It is further contemplated that a structural gene may contain one or more modifications in either the coding segments or in the untranslated regions which could affect the biological activity or the chemical structure of the expression product, the rate of expression or the manner of expression control. Such modifications include, but are not limited to, insertions, deletions, and substitutions of one or more nucleotides. The structural gene may constitute an uninterrupted coding sequence or it may include one or more introns, bounded by the appropriate splice junctions functional in plants. The structural gene may be a composite of segments derived from one or more sources, naturally occurring or synthetic. That structural gene may also produce a fusion protein. In this application a structural gene is considered to include a polyadenylation signal downstream from the translation termination codon. That polyadenylation signal usually results in the addition of polyadenylic acid tracts to the 3'-ends of the precursor mRNAs. It is also known that a canonical polyadenylation signal may cause a cleavage of the transcript and not polyadenylation per se (C. Montell et al. (1983) Nature 305:600). It is contemplated that the introduction into plant tissue of recombinant DNA molecules containing the universal stress-inducible promoter/structural gene complex (the expression complex) will include constructions wherein the structural gene and the regulatory element are not derived from the same source and constructions wherein additional copies of naturally occurring genes are transcribed under the regulatory control of the stress-inducible Gmhsp26-A promoter. It is understood in the art how to combine the requisite functional elements to achieve expression in plant tissue.

Regulatory control refers to the modulation of gene expression by sequence elements upstream of the transcription start site. Regulation may result in an on/off switch for transcription, or it may result in variations in the levels of gene expression. To place a structural gene under regulatory control of sequence elements means to place it sufficiently close to such sequence elements, and in a position relative to such sequence elements so that the gene is switched on or off, so that its level of expression is measurably varied, as is understood by those skilled in the art. There can also be sequence components in the untranslated leader region of mRNA which contribute to the regulation of gene expression at the translational level.

Chemically synthesized, as related to a sequence of DNA, means that the component nucleotides were assembled in vitro using nonenzymatic means. Manual chemical synthesis of DNA may be accomplished using well established procedures (i.e. M. Caruthers (1983) in Methodology of DNA and RNA Sequencing, Weissman (ed.), Praeger Publishers (New York) Chapter 1), or automated synthesis can be performed using one of a number of commercially available machines.

Plant tissue is composed of plant cells and includes differentiated and undifferentiated tissues of plants including, but not limited to, roots, shoots, leaves, pollen, seeds, tumor tissue, such as crown galls, and various forms of aggregations of plant cells in culture, such as embryos and calli. The plant tissue may be in planta or in organ, tissue, or cell culture.

Homology as used herein, refers to identity of nucleotide sequences. The extent of homology between DNA sequences can be empirically determined in DNA hybridization experiments, such as those described in B. Hames and S. Higgins (1985) Nucleic Acid Hybridisation, IRL Press, Oxford, UK.

The Gmhsp26-A gene of Glycine max (soybean) is a heat shock gene, which is so named because it is predicted to encode a protein of about 26 kDa. Unlike other known plant heat shock genes, the transcription of Gmhsp26-A is induced by a wide range of environmental stresses in addition to heat shock. The nucleotide sequence of the Gmhsp26-A gene is given in Figure 2. The gene comprises about 1190 bp of 5'-flanking sequence, a 72 bp transcribed leader region, an open reading frame of 225 codons interrupted by an intron of 388 bp, and 236 bp of 3' downstream transcribed sequence. The function of the gene product is not known.

The induction patterns of several soybean heat shock genes were studied by E. Czarnecka et al. (1984) Plant Mol. Biol. 3:45. Radiolabelled cDNA-containing recombinant plasmids were used as probes against polyadenylated control and stress RNAs in northern hybridization experiments. Table 1 gives a comparison of expression under control cultural conditions and under the stress conditions tested. The heat shock gene of pCE54 (Gmhsp26-A) was chosen for further study because it was induced to higher expression levels in response to a wide variety of environmental stresses. It was also found that there was detectable constitutive expression of this gene.

Table 1. Summary of stress-induced accumulation of poly(A) RNA homologous to heat shock clones of soybean[1,2]

| Stress | pFS2005 | pFS2019 | pFS2033 | pCE75 | pCE53 | pCE54 |
|---|---|---|---|---|---|---|
| Control | – | – | – | – | – | 1+ |
| Heat shock, 40°C | 10+ | 8+ | 5+ | 5+ | 10+ | 5+ |
| Ethylene 10 ppm | – | – | NA | – | – | – |
| 2,4-D 510 μg/ml | 1+ | 1+ | NA | 1+ | 1+ | 5+ |
| $GA_2$ 1mM | – | – | NA | – | – | 2+ |
| ABA 0.75 mM | – | – | NA | – | – | 5+ |
| Polyethylene glycol -8 bars | – | – | NA | – | – | 4+ |
| KCl 125 mM | – | – | NA | – | – | 4+ |
| Arsenite 100 mM | 6+ | 6+ | 2+ | 4+ | 7+ | 5+ |
| Dinitrophenol 1 mM | 1+ | 1+ | NA | 1+ | T | 5+ |
| Anaerobiosis ($N_2$) | T | T | NA | NA | NA | 2+ |
| NaF 1mM | – | 1+ | NA | – | T | 4+ |
| NaCN 0.1mM | – | – | NA | – | T | – |
| Canavanine 0.1 mM | T | T | NA | T | 1+ | 5+ |
| p-F-phenylalanine | – | – | NA | – | – | 3+ |
| Azetidine-2-COOH 0.5mM | 3+ | 2+ | NA | NA | NA | 4+ |
| $CdCl_2$ 0.5 mM | 4+ | 3+ | 1+ | 2+ | 5+ | 6+ |
| $CoCl_2$ 0.25 mM | – | – | NA | – | T | 1+ |
| $AgCl_2$ 0.25 mM | T | T | NA | T | 1+ | 1+ |
| $NiCl_2$ 0.25 mM | – | – | NA | – | – | 1+ |
| $CuCl_2$ 0.25 mM | 2+ | 1+ | NA | 1+ | 1+ | 1+ |

[1] Some data from Czarnecka (1984) Plant Mol. Biol. 3:45-58.

[2] – = not detected;
NA = not analyzed;
+ = estimated relative level from northern hybridization analysis;
T = trace

Sequences homologous to the soybean insert DNA in pCE54 were selected from a genomic recombinant library using conventional DNA hybridization methods. An approximately 6.2 kb BglII fragment was selected and was subsequently found to carry the Gmhsp26-A gene. The restriction map of this 6.2 kb BglII fragment is presented in Figure 1. The nucleotide sequence of the Gmhsp26-A gene was determined. The sequence extending from nucleotides -1190 to +1573 is displayed in Figure 2. Nucleotide sequence analysis confirms that the entire gene was cloned. The DNA sequence comprises 1190 of 5'-flanking sequence, 1335 bp of transcribed DNA, and about 230 bp downstream of the 3'-termini of the transcript. The open reading frame in the transcribed region extends for 225 codons, and is predicted to encode a 26.0 kDa protein. A single intron splits the protein coding region between codons 107 and 108. The donor and acceptor splice sites fit the typical eukaryotic consensus (R. Padgett et al. (1986) Ann. Rev. Biochem. 55:1119).

The end points for transcription of the Gmhsp26-A gene have been studied by S1 nuclease mapping and by reverse transcriptase primer extension experiments. There were found to be one major and two minor start sites for transcription, which are shown in Figure 2. The pattern of protected hybrids indicated the presence of multiple 5'-termini with induction by heat shock and cadmium chloride treatments. These observations were confirmed by the primer extension studies. Those three initiation sites are spaced at about 65 bp intervals and are positioned 27 to 30 bp downstream from sequences similar to TATA consensus regions (indicated in Figure 2). The major, or most abundant, transcript is the one which starts closest 5′ to the ATG which serves as the start site for protein synthesis; the start site of the major transcript is defined as beginning at +1, and the TATA box and ancillary sequences associated with it are called subpromoter 1. The numbering for the two other subpromoters and start sites gives positions relative to the start site for subpromoter 1. The TATA-homologous and other identified sequences associated with subpromoter 2 are those next upstream from subpromoter 1, and the subpromoter furthest 5′ from +1 is called subpromoter 3. Computer-assisted inspection of the nucleotide sequence reveals an AT-rich region resembling the TATA box consensus sequence about 27-31 bp upstream from each transcription start site. The TATA-like motifs are 5′-TATAAAAG-3′, for subpromoter 1, 5′-TATATAGGTATAT-3′ for subpromoter 2, and 5′-AATTAATA-3′ for subpromoter 3.

The 5′-untranscribed region was also analyzed for sequences homologous to the consensus heat shock element (HSE) sequence 5′-CTxGAAxxTTCxAG-3′ (x is A,T,C, or G) (H. Pelham (1982) Cell 30:517). Several overlapping copies of sequences with partial homology to this consensus sequence were found upstream of each of the three putative TATA boxes. The extents of homology to the consensus sequence range from 5/10 to 7/10 matches. In the DNA sequence between -720 and -21 there are ten sequences with at least 50% homology to the HSE consensus sequence, and there is an additional motif with 70% homology to the HSE consensus between +1 and +72. The positions of these HSE-like sequences are displayed in Figure 2. In animal systems, generally poor matches give poor heat shock response, but there is evidence that repetition of the HSE motif compensates for the mismatches to the consensus sequence.

The 5′-flanking sequence of the Gmhsp26-A gene was analyzed for regions homologous to the metal regulatory element (MRE) consensus sequence 5′-CPyTTTGCPuPyPyPyCG-3′ (Pu is A or G, Py is C or T) found in animals (G. Stuart et al. (1984) Proc. Nat. Acad. Sci. USA 81:7318). Several regions of limited homology were found. There is a 7/12 match overlapping the HSE-like sequence of subpromoter 1 while subpromoters 2 and 3 each contain an 8/12 match to the MRE consensus between the TATA motifs and the transcription start sites. MRE-like sequences may function in the heavy metal-regulation of genes of plants. These sequences can represent sites for binding of regulatory factors to DNA.

The relative inducibility of each of the three subpromoters was examined under control growth conditions and under heat shock and cadmium stress conditions. The data of Table 2 show that subpromoter 3 is the least used, but the most inducible under the stress conditions tested. Transcripts from subpromoter 2 are intermediate in abundance. Transcripts originating from subpromoter 1 are the most abundant, but the fold-induction under heat or heavy metal stresses is less than that for subpromoter 3. The relative inducibility of transcription from each of the subpromoters differs depending on the stress treatment, suggesting that each subpromoter represents a specialization towards a specific set of stress conditions. The existence of multiple start sites for the Gmhsp26-A gene suggests that there are multiple transcription factors involved in the induction of the gene under different stress conditions.

Table 2.

Percent of Gmhsp26-A transcription initiation at each of the three major start sites under control and selected stress conditions.

| | % Initiation at each site | | | Fold Induction | |
|---|---|---|---|---|---|
| Start Site | 28°C | 40°C | CdCl$_2$ | 40°C | CdCl$_2$ |
| 3 | 7 | 23 | 18 | 73 | 38 |
| 2 | 34 | 25 | 32 | 16 | 13 |
| 1 | 59 | 50 | 50 | 19 | 12 |

The functionality of any DNA sequences within the plant universal regulatory element can be tested by those skilled in the art of plant molecular biology. It will be understood that there may be minor variations within sequences utilized or disclosed in the present application. It is, however, well known in the art that some DNA sequences within a larger stretch of sequence are more important than others in determining functionality. A skilled artisan can test allowable variations in sequence by mutagenic techniques which include, but are not limited to, those discussed by D. Shortle et al. (1981) Ann. Rev. Genet. 15:265; M. Smith (1985) ibid. 19:423; D. Botstein and D. Shortle (1985) Science 229:1193; S. McKnight and R. Kingsbury (1982) Science 217,316; R. Myers et al. (1986) Science 232:613. It is also known how to generate and analyze deletions of varying lengths (e.g. T. Maniatis et al. (1982) Molecular Cloning, Cold Spring Harbor Laboratory, Cold Spring Harbor, New York). These variations and others can be determined by standard techniques to enable those of ordinary skill in the art to manipulate and bring into utility the functional units of regulatory element and structural gene (see Example 7).

Production of genetically modified plant tissue expressing a structural gene under the transcriptional control of a stress-inducible promoter functional in plants combines the specific teachings of the present disclosure with a variety of techniques and expedients known in the art. In most instances, alternative expedients exist for each stage of the overall process. The choice of expedients depends on such variables as the choice of the vector system for the introduction and stable maintenance of the expression complex, the plant species to be modified and the desired regeneration strategy, and the particular structural gene to be used. Those of ordinary skill are able to select and use appropriate alternative process steps to achieve a desired result. For instance, although an ultimate starting point for obtaining the plant regulatory element of the present invention is the Gmhsp26-A gene of Glycine max var. Corsoy, exemplified in the present application, homologous DNA sequences of other plant stress genes, or from different sources, can be substituted as long as the appropriate modifications are made to the procedures for manipulating the DNA carrying the plant universal stress-inducible regulatory element, and provided it is known that the regulation afforded by the alternative sequences is equivalent. Similarly, the Gmhsp26-A structural gene can be replaced by any plant-expressible structural gene, again with appropriate procedural modifications. Homologs of structural genes or of other sequences may be identified by the ability of their nucleic acids to cross-hybridize under conditions of appropriate stringency as is well understood in the art.

A principal feature of the present invention is a recombinant DNA molecule having a plant-expressible gene whose transcriptional expression is controlled by a universal stress-inducible regulatory element, such as that of the Gmhsp26-A gene of soybean. The expression complex comprises the promoter and promoter-associated sequences of the universal stress-inducible regulatory element and a structural gene expressible in a plant. The promoter sequences and structural gene must be correctly positioned and oriented relative to one another such that the promoter sequences can activate transcription of the structural gene. To be controlled by the stress-inducible regulatory element, the structural gene must be inserted on the 3'-side of said regulatory element so that 5'-end of the gene is adjacent to the 3'-end of the regulatory element. A polyadenylation signal must be located in the correct orientation downstream from the 3'-end of the coding sequence. Another consideration is the distance between the functional elements of the expression complex. Substantial variation appears to exist with regard to these distances; therefore, the distance requirements are best described in terms of functionality. As a first approximation, reasonable operability can be obtained when the distances between functional elements are similar to those in the genes from which they were derived. The distance between the promoter sequences and the 5'-end of the structural gene, or between the upstream promoter associated sequence elements which are responsible for regulatory control and other components in the construction can be varied, and thus one can achieve variations in the levels of expression of the downstream structural gene. In the case of constructions yielding fusion proteins, an additional requirement is that the ligation of the two genes or fragments thereof must be such that the two coding sequences are in the same reading frame, a requirement well understood in the art. An exception to this requirement exists in the case where an intron separates the coding sequence derived from one gene from the coding sequence of the other. In that case, the coding sequences must be bounded by compatible splice sites, and the intron splice sites must be positioned so that the correct reading frame for both genes is established in the fusion after the introns are removed by post-transcriptional processing. It is generally understood in the art how to achieve gene expression in plants, and the skilled artisan will ensure that all necessary requirements are met.

The recombinant DNA molecule carrying the desired structural gene under the control of the stress-inducible promoter, for example that from the Gmhsp26-A gene of soybean, may be introduced into plant tissue by any means known to those skilled in the art. The technique used for a given plant species or specific type of plant tissue depends on the known successful techniques. As novel means are developed for the stable insertion of foreign genes into plant cells and for manipulating the modified cells, skilled artisans will be able to select from known means to achieve a desired result. Means for introducing recombinant DNA into plant tissue include, but are not limited to transformation (J. Paszkowski et al. (1984) EMBO J. 3:2717), electroporation (M. Fromm et al. (1985) Proc. Natl. Acad. Sci. USA 82:5824), microinjection (A. Crossway et al. (1986) Mol. Gen. Genet. 202:179), or T-DNA mediated transfer from Agrobacterium tumefaciens to the plant tissue. There appears to be no fundamental limitation of T-DNA transformation to the natural host range of Agrobacterium. Successful T-DNA-mediated transformation of monocots (G. Hooykaas-Van Slogteren et al. (1984) Nature 311:763), gymnosperm (A. Dandekar et al. (1987) Biotechnol. 5:587) and algae (R. Ausich EPO application 108,580) has been reported. Representative T-DNA vector systems are described in the following references: G. An et al. (1985) EMBO J. 4:277; L. Herrera-Estrella et al. (1983) Nature 303:209; L. Herrera-Estrella et al. (1983) EMBO J. 2:987; L. Herrera-Estrella et al. (1985) in Plant Genetic Engineering, New York: Cambridge University Press, p.63. Once introduced into the plant tissue, the expression of the structural gene may be assayed by any means known to the art, and expression may be measured as mRNA transcribed or as protein synthesized. Techniques are known for the in vitro culture of plant tissue, and in a number of cases, for regeneration into whole plants. Procedures for transferring the introduced expression complex to commercially useful cultivars are known to those skilled in the art.

In a specific embodiment the Gmhsp26-A gene has been inserted in the T-DNA based shuttle vector pW9 which will allow the transfer of the Gmhsp26-A gene to heterologous plant hosts. As will be readily apparent to those of ordinary skill in the art, other plant-expressible genes can be incorporated in place of the Gmhsp26-A coding region of the expression complex using any naturally occurring or artificially engineered restriction sites convenient for in vitro manipulations. The major consideration is that the sequences at the junctions remain compatible with transcriptional and translational functionality. The final steps of the preferred embodiment for obtaining genetically modified plant tissue include inserting the expression complex into a T-DNA-containing vector, and transferring the recombinant DNA to plant tissue wherein the modified T-DNA becomes stably integrated as part of the genome.

The following examples are provided for illustrative purposes only and are not intended to limit the scope of the invention. The examples utilize many techniques well known and accessible to those skilled in the arts of molecular biology, in the manipulation of recombinant DNA in plant tissue, and in the culture and regeneration of transformed plants. Enzymes are obtained from commercial sources and are used according to the vendors' recommendations or other variations known in the art. Reagents, buffers and culture conditions are also known to the art. References containing standard molecular biological procedures include T. Maniatis et al. (1982) Molecular Cloning, Cold Spring Harbor Laboratory, Cold Spring Harbor, New York; R. Wu (ed.) (1979) Meth. Enzymol. 68; R. Wu et al. (eds.) (1983) Meth. Enzymol. 100 and 101: L. Grossman and K. Moldave (eds.) (1980) Meth. Enzymol. 65; J. Miller (ed.) (1972) Experiments in Molecular Genetics, Cold Spring Harbor Laboratory, Cold Spring Harbor, New York; Old and Primrose (1981) Principles of Gene Manipulation, University of California Press, Berkeley, California; R. Schlief and P. Wensink (1982) Practical Methods in Molecular Biology; Glover (ed.) (1985) DNA Cloning, Vols. I and II, IRL Press, Oxford, UK; Hames and Higgins (eds.) (1985) Nucleic Acid Hybridisation, IRL Press, Oxford, UK; Setlow and A. Hollaender (1979) Genetic Engineering: Principles and Methods, Vols. 1-4, Plenum Press, New York, which are expressly incorporated by reference herein. Abbreviations and nomenclature, where employed, are deemed standard in the field and commonly used in professional journals such as those cited herein.

## Example 1: Isolation of a genomic clone of the Gmhsp26-A gene

This example briefly describes the steps leading to the isolation of the genomic clone of the Gmhsp26-A heat shock gene of Glycine max var. Corsoy.

## Example 1.1 Isolation of cDNA clones homologous to stress-induced polyadenylated RNAs

A cDNA library was constructed using polyadenylated RNA from heat-shocked soybean hypocotyls as template for reverse transcriptase (F. Schoffl and J. Key (1982) J. Mol. Appl. Genet. 1:301).pFS2005, pFS2019, and pFS2033 are three recombinant plasmids carrying cDNA copies of heat-inducible transcripts isolated in this study.

Subsequently, E. Czarnecka et al. (1984) Plant Mol. Biol. 3:45, isolated additional cDNA clones corresponding to stress-inducible transcripts. Control and stress-induced polyadenylated RNA was isolated from soybean seedlings or cultured slices of mature hypocotyl. Stress treatments included heat shock at 40°C, salt stress with 125 mM potassium chloride, low water potential with -5 bars polyethylene glycol-6000, plant growth regulators at 0.75 mM abscisic acid or 510 µg/ml 2,4-dichlorophenoxyacetic acid, inhibition of respiration with 0.1 mM sodium arsenite or 1 mM dinitrophenol, synthesis of defective protein with amino acid analogues using 0.1 mM canavanine or 1 mM p-fluorophenylalanine, sparging with ethylene at 10 ppm in air, and anaerobiosis resulting from sparging of the incubation medium with nitrogen gas. Radiolabelled cDNAS were produced from the poly(A) + RNA samples, and were used to screen the cDNA library described above. pCE53, pCE54, and pCE75 are clones which showed an increase in hybridization signals when screened with cDNAS from several stress conditions as compared with the control signals. pCE54 had a detectable level of expression under control conditions, and was induced under most of the stress conditions tested (E. Czarnecka et al. (1984)).

## Example 1.2 Expression of heat-inducible genes in response to environmental stress and under normal cultural conditions

pCE54, pCE53, pCE75 and the three heat-inducible clones selected by Schoffl and Key (1982) were radiolabelled for use in northern hybridization experiments to analyze response patterns of genes induced by environmental stress(es) (E. Czarnecka et al. (1984)). pCE54 was chosen for further study because of its unique pattern of expression. The gene homologous to this probe was expressed constitutively, and was induced under nearly all stress conditions tested. It has been found that the level of apparent constitutive expression observed with pCE54 depends on the treatment of tissue samples. Control tissue samples that are immediately frozen in liquid nitrogen after excision show much lower levels of gene expression than tissues which are excised and cooled on ice before assay. This indicates that wounding of the tissue and possibly cold shock (cooling on ice) are stimuli which induce expression of the Gmhsp26-A gene.

### Example 1.3 Isolation of a genomic clone homologous to pCE54

pCE54 DNA was radiolabelled for use as a hybridization probe in screening a lambda 1059 recombinant library of soybean genomic DNA constructed by J. Slightom and Y. Ma, Agrigenetics Advanced Research Laboratory, Madison, Wisconsin. Total soybean (Glycine max var. Corsoy) DNA was partially digested with MboI and ligated into the BamHI site of lambda 1059 (J. Karn et al. (1980) Proc. Nat. Acad. Sci. USA 77:5172). Previous use of this library was described by R. Nagao et al. (1985) Mol. Cell. Biol. 5:3417. The screening of the present work yielded hsY54 as a recombinant plasmid hybridizing to pCE54. BglII digestion yielded three major insert fragments of approximately 4, 6, and 9 kb. Hybridization analysis determined that the soybean-specific insert of pCE54 hybridized to 4 and 6.2 kb BglII restriction fragments in hsY54. A restriction map of the approximately 6.2 kb BglII fragment is presented in Figure 1.

### Example 2: DNA sequence determination of the Gmhsp26-A gene

The 6.2 kb BglII of hsY54 was subcloned into pUC vectors (J. Vieira and J. Messing (1982) Gene 19:259).

Clones containing overlapping deletions for sequencing were constructed by the method of R. Dale et al. (1985) Plasmid 13:31. The DNA sequence of the soybean insert was subsequently determined using the chemical cleavage technique of A. Maxam and W. Gilbert (1980) Meth. Enzymol. 65:499, and the dideoxynucleotide chain termination method of F. Sanger et al. (1980) J. Mol. Biol. 143:161. Sequences from -420 to +1573 were determined for both strands, while only the sense strand was sequenced for the far upstream region (-1190 to -420). Inspection of the sequence revealed that the entire Gmhsp26-A gene was contained within the 6 kb BglII fragment of hsY54. The sequence from -1190 to +1573 is given in Figure 2.

### Example 3: Transcript mapping of the Gmhsp26-A gene

Transcripts homologous to Gmhsp26-A were mapped either by S1 nuclease protection using 1 to 2 µg of poly(A) RNA or by primer extension using 5 to 10 µg of poly(A) RNA. S1 nuclease mapping of transcripts was performed according to the procedure of J. Favaloro et al. (1980) Methods Enzymol. 65:718 as described previously (E. Czarnecka et al. (1985) Proc. Nat. Acad. Sci. USA 82:3726). The hybridization probe used for the analysis of the 5'-termini of the transcripts of the Gmhsp26-A gene was the 1.6 kb HindIII-SalI fragment labelled at the 5'-end HindIII site (within the first exon).

Reverse transcriptase primer extension analysis was performed using a synthetic oligonucleotide (5'-GGAACAAGTATAGCTGG-3') primer complementary to sequences downstream from the 5'-termini at positions +24 to +40. Control, heat-shocked, or cadmium-induced poly(A) RNA was annealed with 5'-end-labelled primer at room temperature for 16 h in sealed microcapillaries. The extension reaction mixtures were incubated with 200 units of Maloney MLV reverse transcriptase (Bethesda Research Laboratories) for 1 h at room temperature. The in vitro synthesized products were separated on 8% polyacrylamide-urea gels and exposed with screens at -70°C. The percentage of total Gmhsp26-A transcription under different conditions originating from each of the three major start sites was determined by counting radioactivity from isolated bands cut from the S1 nuclease mapping gel.

### Example 4. Construction of pGmhsp26-A

This example describes the strategy which is employed to express a plant-expressible structural gene, such as the Gmhsp26-A heat shock gene of soybean, under the regulatory control of the plant universal stress-inducible regulatory element of the soybean Gmhsp26-A gene.

### Example 4.1 Construction of pGmhsp26-A26-A

The Gmhsp26-A gene of Glycine max var. Corsoy is isolated from the lambda genomic clone hsY54 as a SalI-PstI fragment of approximately 3.2 kb. After modification of this fragment by the addition of BglII linkers, the fragment is ligated into the BglII-cut vector plasmid pW9 described previously (W. Gurley et al. (1986) Mol. Cell. Biol. 6,559). The DNA sequences derived from T-left of pTi15955 provide the site for the homologous recombination of the shuttle vector into the Ti plasmid of the Agrobacterium tumefaciens intermediate host. The Agrobacterium host effects the transfer of the Gmhsp26-A expression complex to plant tissue for subsequent induced expression under the regulatory control of environmental stress.

### Example 5. Vector transfer and tumor formation

A. tumefaciens 5260 (W. Gurley et al. (1987) Mol. Cell. Biol. 7:59) is used as the recipient for shuttle vectors containing the various constructions containing Gmhsp26-A derivatives or chimeric constructions. It is from this strain that the recombinant molecules are transferred to plant tissue.

Double gene shuttle vectors are transferred from E. coli LE392 into A. tumefaciens by triparental conjugation on solid nutrient medium as described by R. Fraley et al. (1983) Proc. Nat. Acad. Sci USA 80:4803. The resulting transconjugant colonies are selected by growth on AB minimal medium (M. Chilton et al. (1974) Proc. Natl. Acad.

Sci. USA 71:3672) containing streptomycin (250 µg/ml) and kanamycin (20 µg/ml) for 3 to 5 days at 28°C. Tumors are incited on sunflower (Helianthus annuus. cv. Large Grey) seedlings, and the plants are grown as previously described (W. Gurley et al. (1986) 6:559). After 14 to 16 days of tumor growth, an average of 200 to 300 tumors for each plasmid construction are harvested and immediately frozen with liquid nitrogen for analysis of constitutive expression.

Example 6: Stress-induced gene expression

Tumor tissue is incubated in growth medium at 28°C for control constitutive gene expression or at 40°C for heat shock-induced expression. Conditions of other forms of environmental stress are as described in Czarnecka et al. (1984). Tissue is harvested for extraction of RNA for analysis by northern hybridization analysis as described above.

Example 7: Cloning of Gmhsp26-A for functional analysis of promotor sequences

The BglII fragment (pGmhsp26A, Example 4) is isolated, the ends are made blunt by treatment with Klenow DNA polymerase and the fragment is then inserted into the SmaI site of pUC19. The pUC19 derivative is then manipulated to make deletions and other sequence modifications in the upstream region of the gene employing techniques well-known to those in the art (T. Maniatis et al (1982); D. Shortle et al. (1981); M. Smith (1985); D. Botstein and D. Shortle (1984); McKnight and Kingsbury (1982); Myers et al. (1986)). Mutant derivatives of the plant universal stress-inducible regulatory element can be tested in a plasmid system such as pMH1 (L. Herrera-Estrella et al. (1984) Nature 310:115). pMH1 carries a chloramphenicol acetyl transferase structural gene which can be used as a reporter gene to monitor the functionality of regulatory and promoter sequences inserted upstream therefrom. pMH1 also carries DNA sequences with homology to the Ti plasmid of Agrobacterium so that it is possible to transfer the expression complex to plant tissue, where the functionality of derivatives of the regulatory element can be measured in response to application of stress.

**Claims**

1. A recombinant DNA molecule comprising a plant universal stress-inducible regulatory element and a plant-expressible structural gene positioned such that said structural gene is placed under the regulatory control of said plant universal stress-inducible regulatory element.

2. The recombinant DNA molecule of claim 1 wherein said plant universal stress-inducible regulatory element is that of the Gmhsp26-A gene of soybean, or has at least about 90% nucleotide sequence homology thereto.

3. The recombinant DNA molecule of claim 1 wherein said plant universal stress-inducible regulatory element comprises the nucleotide sequence as in Figure 2, from a start point at about nucleotide -720 to an end point selected from any of about -21, nucleotide +1 or nucleotide +72, or a nucleotide sequence at least about 90% homologous thereto.

4. The recombinant DNA molecule of claim 1 wherein said plant universal stress-inducible regulatory element comprises the nucleotide sequence as in Figure 2, from a start point at about nucleotide -1190 to an end point selected from any of about nucleotide -21, nucleotide +1 or nucleotide +72, or a nucleotide sequence at least about 90% homologous thereto.

5. The recombinant DNA molecule of claim 1 wherein said plant universal stress-inducible regulatory element comprises a copy of a plant heat shock element wherein said plant heat shock element comprises a nucleotide sequence with 50% or greater homology to the consensus heat shock element sequence 5-CTxGAAxxTTCxAG-3'.

6. The recombinant DNA molecule of claim 1 wherein said plant-expressible structural gene is not a plant heat shock structural gene.

7. A method for inducing an increased level of expression of a plant-expressible gene in a plant tissue in response to conditions of environmental stress comprising the steps of: introducing into plant tissue a recombinant DNA molecule comprising a plant universal stress-inducible regulatory element and a plant-expressible structural gene positioned such that said plant expressible structural gene is expressed under the regulatory control of said plant universal stress-inducible regulatory element in said plant tissue; and applying stress to said plant tissue such that said regulatory element induces expression of said structural gene.

8. The method of claim 12 wherein said plant universal stress-inducible regulatory element is that of the Gmhsp26-A gene of soybean, or has at least about 90% nucleotide sequence homology thereto.

9. The method of claim 12 wherein said plant universal stress-inducible regulatory element comprises the nucleotide sequence as in Figure 2 from a start point at about nucleotide -720 to an end point selected from any of about nucleotide -21, nucleotide +1 or nucleotide +72, or a nucleotide sequence at least about 90% homologous thereto.

10. The method of claim 12 wherein said plant universal stress-inducible regulatory element comprises the nucleotide sequence as in Figure 2, from a start point at about nucleotide -1190 to an end point selected from any of about nucleotide -21, nucleotide +1 or nucleotide +72, or a nucleotide sequence having at least about 90% homology thereto.

11. The method of claim 12 wherein said plant universal stress-inducible regulatory element comprises a copy of a plant heat shock element, wherein said plant heat shock element has a nucleotide sequence with 50% or greater homology to the consensus heat shock element sequence 5'-CTxGAAxxTTCxAG-3'.

12. The method of claim 12 wherein said plant-expressible structural gene is not a plant-heat shock gene.

# FIG. 1

**Gmhsp26-A**
**Heat Shock-Stress Gene**

Bgl II      Hinc II    +1    Hinc II      Bgl II

EcoR1
Hind III
Sal I
Pst I
Bam H1

EP 0 330 479 A2

1 kb

cDNA pCE54

primary transcript

5'          3'

intron

▨ = coding region
■ = untranslated 5' or 3'

# FIG. 2-1

```
-1190    -1180      -1170      -1160      -1150      -1140      -1130      -1120      -1110      -1100      -1090      -1080
CCATACAAACAATACATAACTAAATAACTGTAATTTTATGTTTTTCTTTCATTGTATTGTGCGTTTTATATTAATGTATTAATTATGTTGTTTTTTTAATTTTTATTGATAAATTATTTAAA

-1070 d  -1060      -1050      -1040      -1030      -1020      -1010      -1000      -990       -980       -970       -960
CTTTAAATAAAAAACATTTAAAGTAAACTATAATAAAAATATATATAACATGTTTTAAATAATTAAACATTAAAATAAAATAATAACATATCAAATAAAATCACAAAAAAATATACTAATG

-950     -940       -930       -920       -910       -900       -890       -880       -870       -860       -850       -840
AAATCAACTTAATATTATTTATTATATATTTAAATATATATATTTTTTAACTTTTTTTATTTTAAAAAAATAAAATTGTTAACATTATAAAATTAAAAAACAAATTTATGAAAAAAAAGGTTA

-830     -820       -810       -800       -790       -780       -770       -760       -750       -740       -730       -720  7    6
ACAAATTAAAAACAAACTACAAATAGTAAAATAACGTTTTTAAAAAAAAAAGGAGGTTACGATCTCAAAATCGTATACCTCATGAAATTTTTAAAAAATAAGTAAGACAGTTTTACAGCT

-710 d   -700       -690       -680       -670 d     -660       -650       -640       -630       -620       -610       -600
TTAAGTAATAAAACAAAATACGAGTTTTTTTTTTAATACGACTTTAAAGTCGTAAAAAAATTATATTTACATTGAAGTTGTAAATAATATTTTTACGATTTCAGTTAAAAAAATAAGAAA

-590     -580       -570       -560       -550       -540       -530       -520       -510       -500       -490       -480
ACTAAAAAGTCGTAATAGGTGCTAACAACTGTTACAACTTAACCCGTTTTAGATAATTTTTTTAAAATATACCCCAAATGAAAAATTGCGGATTATTTTATCCCCAATTGGTAAAAAAGCA

-470     -460       -450       -440       -430       -420       -410       -400       -390       -380       -370       -360
CCTGAATACTAGAAACAAGGGTGGCAAAGATTCAAAGTAATGAATTTATAAAAAAAAAAAGAAAATATTTATATAATTACACAAAATCTTAAAAAAATGTTGTTATTATTTACTACTAATT

-350  6  -340   7  -330       -320       -310       -300       -290       -280       -270       -260       -250       -240
TATAATTCGAACGAGTATATTTAAGCAAAGTTCTCTACGCGGTTTACGTAATCTCTTACATCATTTATTTTTCCACGTTTGTTTACTCTGAGGCATCTTCTTGATAGGCGAAAGTTTT

-230  6  -220   7  -210       -200       -190       -180       -170       -160       -150  6   -140       -130       -120
TTCATATTTTTTCTACATOCTTCTCATGTGCAATGTCTCCGCATTCATACGCAGCAATCAAAATGGAATTAATACACGTAGCACTTTCCCATTTTTTTGTTATCGTCCCCACTATTGAC
                                                                          3              5'
```

EP 0 330 479 A2

# FIG. 2-3

```
        850           860           870           880           890       7   900           910           920           930
GTT GAA GAA ACA TAT GAG GCT CTT CAG TTT CTT GAG AAT GAG CTG AAG GAC AAG AAG TTT TTT GGA GGA GAG GAA TTT GGG TTG GTA GAT
Val Glu Glu Thr Tyr Glu Ala Leu Gln Phe Leu Glu Asn Glu Leu Lys Asp Lys Lys Phe Phe Gly Gly Glu Glu Phe Gly Leu Val Asp

        940           950           960           970           980           990          1000          1010          1020
ATT GCT GCT GTC TTC ATA GCA TTT TGG ATC CCA ATT TTT CAG GAA ATA GCA GGG TTG CAG TTA TTC ACC AGT GAG AAA TTT CCT ATA CTC
Ile Ala Ala Val Phe Ile Ala Phe Trp Ile Pro Ile Phe Gln Glu Ile Ala Gly Leu Gln Leu Phe Thr Ser Glu Lys Phe Pro Ile Leu

       1030          1040          1050          1060          1070          1080          1090          1100          1110
TAC AAA TGG AGC CAA GAA TTC CTT AAC CAC CCT TTT GTG CAC GAA GTC CTT CCT CCT AGA GAC CCA CTT TTT GCC TAC TTC AAA GCC CGC
Tyr Lys Trp Ser Gln Glu Phe Leu Asn His Pro Phe Val His Glu Val Leu Pro Pro Arg Asp Pro Leu Phe Ala Tyr Phe Lys Ala Arg

       1120          1130          1140          1150          1160          1170          1180          1190          1200          1210          1220
TAT GAA AGT CTT TCT GCT TCA AAA TAG ACTTATTTAAGGATATTTGTTGAACAACTTGTGTCTTGTTGAGTTATTGCTGTTTGAATTTCATGTAAAATGATACTAGCTATA
Tyr Glu Ser Leu Ser Ala Ser Lys ——

       1230          1240          1250          1260          1270          1280          1290          1300          1310          1320          1330          1340
TGTAAATCCCAGAAAAAAAAAAAAAAGAATCCTAGGATCTTGTTTTCGTTTTGGCCATTTCAGTTAATAAAGAAATTATATTTTTCGATATAAATTTTGTTGTGAAAGCTTTATTCTTC
                                                                                                                              3'          3'

       1350          1360          1370          1380          1390          1400 7       1410 7      1420          1430          1440          1450          1460
CTTCATAAAATCCTTCAATGTGCATAATCTTATTCGTAGAGAGACTTAGAGCGCTAGTAGCTACTACTTGAAATTTTTTCTTAATTCGAAGGACAACGTATATATTATATAATAATA

       1470          1480          1490          1500          1510          1520          1530    8 1540          1550          1560          1570
ATTATTGCAAGTTGGAAATCGTGTAAGCATGTTTCATGACTATATGAGTTAACAATATACTGTCTTGCCTGCAACCTTCATCATTCTAAAATTATTCCCTTGGCTGCA
```